# EUROPEAN PATENT APPLICATION

(11) **EP 1 953 150 A1**
(43) Date of publication of application: **06.08.2008**
(21) Application number: 06832924.2
(22) Date of filing: 13.11.2006
(51) Int. Cl.: C07D 401/04, C07B 61/00

(54) **PROCESS FOR PRODUCTION OF 2-(4-METHYL-2-PHENYLPIPERAZIN- 1-YL)PYRIDINE-3-METHANOL**

(30) Priority: 14.11.2005 JP 2005328443
(71) Applicant: Sumitomo Chemical Company, Limited, Tokyo 104-8260 (JP)
(72) Inventor: SUGIHARA, Toru, Nishinomiya-shi, Hyogo 663-8101 (JP); MAEDA, Chiharu, Higashiosaka-shi, Osaka 577-0807 (JP)
(74) Representative: Duckworth, Timothy John
(86) International application number: PCT/JP2006/323047
(87) International publication number: WO 2007/055423

(57) **Abstract**

The present invention provides a method for producing 2-(4-methyl-2-phenylpiperazin-1-yl)pyridine-3-methanol, and this method includes the step of catalytically reducing 2-(4-methyl-2-phenylpiperazin-1-yl)-3-cyanopyridine in the presence of a partially deactivated palladium catalyst in an aqueous acid solution.

## Description

### Technical Field

The present invention relates to a method for producing 2-(4-methyl-2-phenylpiperazin-1-yl)pyridine-3-methanol. More particularly, the present invention relates to a method for producing 2-(4-methyl-2-phenylpiperazin-1-yl)pyridine-3-methanol which is properly used as a production intermediate for mirtazapine useful as an antidepressant.

### Background Art

Mirtazapine is a compound useful as an antidepressant. 2-(4-Methyl-2-phenylpiperazin-1-yl)pyridine-3-methanol is known as a production intermediate for the mirtazapine. As for a method for producing it, the method is known that includes the steps of hydrolysis or alkaline-decomposition of 2-(4-methyl-2-phenylpiperazin-1-yl)-3-cyanopyridine so as to make a carboxylic acid compound, and reduction of the carboxylic acid compound with lithium aluminum hydride (e.g., JP-S59-42678-B, USP4062848).

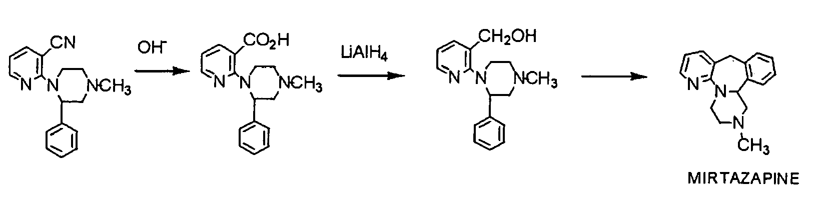

The known methods need a process through the carboxylic acid compound, and further need processes of once drying the carboxylic acid compound in order to reduce it and reducing the carboxylic acid compound under anhydrous conditions. Further, the methods also need 3 moles or more of lithium aluminum hydride which is expensive. Therefore, this method is not sufficient for an industrial production method.

### Disclosure of the Invention

An object of the present invention is to provide an efficient method for producing 2-(4-methyl-2-phenylpiperazin-1-yl)pyridine-3-methanol, which is useful as a production intermediate for mirtazapine, in industrial production.

This object and the other objects are explained by the followings.

Present inventors worked to solve the above-described problems and, as a result, they found out the present invention.

That is, the present invention is as follows.
<1> A method for producing 2-(4-methyl-2-phenylpiperazin-1-yl)pyridine-3-methanol comprising the step of catalytically reducing 2-(4-methyl-2-phenylpiperazin-1-yl)-3-cyanopyridine in the presence of a partially deactivated palladium catalyst in an aqueous acid solution.
<2> The method described in <1>, wherein the partially deactivated palladium catalyst is a palladium catalyst partially deactivated with a compound containing iron.
<3> The method described in <2>, wherein the compound containing iron is iron sulfate, iron chloride or iron acetate.
<4> The method described in any one of <1> to <3>, wherein the aqueous acid solution is an aqueous sulfuric acid solution.
<5> The method described in <4>, wherein the partially deactivated palladium catalyst is prepared by adding a palladium catalyst of 1 to 10 parts by weight to an aqueous solution containing iron sulfate of 0.05 to 0.1 part by weight, stirring the mixture at 0 to 40°C for 1 to 5 hours, filtrating and washing.
<6> The method described in any one of <1> to <5>, wherein the amount of the partially deactivated palladium catalyst is 0.1 to 5 parts by weight as an amount of palladium metal based on 100 parts by weight of 2-(4-methyl-2-phenylpiperazin-1-yl)-3-cyanopyridine.

### Best Mode for Carrying out the Invention

2-(4-Methyl-2-phenylpiperazin-1-yl)-3-cyanopyridine as a starting material in a method of the present invention can be produced by methods described in, for example, JP-S59-42678-B and WO01/023345-A. This compound can be used in any form of a free base and a salt.

Examples of the acid for the aqueous acid solution include sulfuric acid, phosphoric acid and methanesulfonic acid. Sulfuric acid is preferable from the viewpoints of reactivity and economical efficiency.

Further, the aqueous acid solution can be mixed with a solvent such as acetic acid or methanol in an amount not to influence the catalytic reduction.

The acid concentration in the aqueous acid solution is generally 20 to 60% by weight, and preferably 30 to 55% by weight.

The amount of the aqueous acid solution is generally 500 to 2000 parts by volume, and preferably 500 to 1000 parts by volume based on 100 parts by weight of 2-(4-methyl-2-phenylpiperazin-1-yl)-3-cyanopyridine.

The palladium catalyst supported on activated carbon, alumina or zeolite is used. A palladium-carbon catalyst is preferably used from the viewpoints of reactivity and economical efficiency.

The partially deactivated palladium catalyst is also called as a poisoned palladium catalyst, and means a catalyst decreasing activity of the palladium catalyst. A compound (a catalyst poison) capable of acting on the palladium catalyst to prepare the poisoned palladium catalyst is a compound containing iron such as iron sulfate, iron chloride or iron acetate; a compound containing lead such as lead acetate or lead carbonate; or a compound containing bismuth such as bismuth nitrate. The compound containing iron is preferable, and iron sulfate is more preferable.

A method for preparing the palladium catalyst partially deactivated with a catalyst poison includes the steps of, for example, suspending a commercial palladium-carbon catalyst in water and adding an aqueous solution of a compound containing a metal, or adding a palladium-carbon catalyst to an aqueous solution of a compound containing a metal, stirring the mixture for a predetermined time, and filtrating. Further, a palladium catalyst prepared in an aqueous solution of a compound containing a metal can be used.

More particularly, for example, when the catalyst poison is iron sulfate, the partially deactivated palladium catalyst can be prepared by adding a palladium catalyst of 1 to 10 parts by weight to an aqueous solution containing iron sulfate of 0.05 to 0.1 part by weight, stirring the mixture at 0 to 40°C for 1 to 5 hours, filtrating and washing. In a case of the other catalyst poisons, the partially deactivated palladium catalyst can be prepared by a similar process. This preparation can be carried out under an inert gas atmosphere such as nitrogen.

The amount of the partially deactivated palladium catalyst is generally 0.1 to 5 parts by weight as an amount of palladium metal based on 100 parts by weight of 2- (4-methyl-2-phenylpiperazin-1-yl)-3-cyanopyridine.

The temperature in a catalytic reduction is generally 35 to 80°C, and preferably 60 to 70°C.

The hydrogen pressure in a catalytic reduction is generally 1 to 10 kgf/cm² (98 to 980 kPa) as a gage pressure, and preferably 3 to 5 kgf/cm² (294 to 490 kPa).

2-(4-Methyl-2-phenylpiperazin-1-yl)pyridine-3-methanol as an object compound can be obtained by subjecting a reaction liquid after the reduction reaction to general post-treatment operations such as neutralization, extraction, concentration and crystallization.

The obtained 2-(4-methyl-2-phenylpiperazin-1-yl) pyridine-3-methanol can be purified by a method such as recrystallization or silica gel column chromatography if needed.

For example, 2-(4-methyl-2-phenylpiperazin-1-yl) pyridine-3-methanol can be purified by recrystallizing with one or more solvents selected from solvents in an aromatic solvents such as toluene, lower alcohols such as methanol and butanol, and hydrocarbon solvents such as heptane.

The obtained 2-(4-methyl-2-phenylpiperazin-1-yl) pyridine-3-methanol can be used for producing mirtazapine by the methods described in JP-S59-42678-B and the like.

Next, the present invention will be explained in detail by examples, but the present invention is not limited to these

### examples.

### Preparation example of a catalyst:

Six grams (6.0 g) of a palladium-carbon catalyst which was partially deactivated with iron was produced by dissolving 0.035 g of iron sulfate with 50 ml of water under nitrogen atmosphere, adding 5.0 g of 5% palladium-carbon catalyst (54% wet product), stirring at a room temperature for 2 hours, filtrating and washing.

### Example 1

One gram (1 g) of 2-(4-methyl-2-phenylpiperazin-1-yl)-3-cyanopyridine, 10 ml of an aqueous sulfuric acid solution of 50% by weight, and 0.2 g of the catalyst produced by the preparation example of a catalyst were mixed and catalytically reduced in hydrogen at a gage pressure of 3 kgf/cm² (294 kPa) and 70°C. After 5 hours, the reaction liquid was analyzed by a HPLC analysis, and it was confirmed that 71.9% of 2-(4-methyl-2-phenylpiperazin-1-yl)pyridine-3-methanol was produced.

### Example 2

One gram (1 g) of 2-(4-methyl-2-phenylpiperazin-1-yl)-3-cyanopyridine, 5 ml of an aqueous sulfuric acid solution of 50% by weight, and 0.2 g of the catalyst produced by the preparation example of a catalyst were mixed and catalytically reduced in hydrogen at a hydrogen gage pressure of 3 kgf/cm² (294 kPa) and 70°C. After 5 hours, the reaction liquid was analyzed by a HPLC analysis, and it was confirmed that 68.3% of 2-(4-methyl-2-phenylpiperazin-1-yl)pyridine-3-methanol was produced.

### Example 3

One gram (1 g) of 2-(4-methyl-2-phenylpiperazin-1-yl)-3-cyanopyridine, 10 ml of an aqueous sulfuric acid solution of 33% by weight, and 0.2 g of the catalyst produced by the preparation example of a catalyst were mixed and catalytically reduced in hydrogen at a gage pressure of 3 kgf/cm² (294 kPa) and 70°C. The reaction liquid was analyzed by a HPLC analysis, and it was confirmed that 69.1% of 2-(4-methyl-2-phenylpiperazin-1-yl)pyridine-3-methanol was produced. The reaction liquid was neutralized with an aqueous sodium hydroxide solution of 25% by weight and extracted with 30 ml of toluene. The extracted organic layer was concentrated, and 0.4 g of xylene, 0.1 g of 1-butanol and 1.2 g of methanol were added thereto, and the mixture was heated up to 60°C so as to be dissolved. Then, 0.53 g of 2- (4-methyl-2-phenylpiperazin-1-yl)pyridine-3-methanol was produced by adding 1.3 g of heptane to the heated dissolved liquid, cooling down to 0 to 5°C, aging for 1 hour to deposit a crystal, washing the deposited crystal with a mixture of toluene and heptane (solvent ratio was 1:1), and drying the crystal. (Yield: 52%, Purity: 95.8%) IR (KBr) ν = 1573, 1429, 1128. 1036, 757.8, 701 cm⁻¹

### Example 4

One gram (1 g) of 2-(4-methyl-2-phenylpiperazin-1-yl)-3-cyanopyridine, 10 ml of an aqueous sulfuric acid solution of 50% by weight, and 0.2 g of the catalyst produced by the preparation example of a catalyst were mixed and catalytically reduced in hydrogen at a hydrogen gage pressure of 3 kgf/cm² (294 kPa) and 25°C. After 20 hours, the reaction liquid was analyzed by a HPLC analysis, and it was confirmed that 20.9% of 2-(4-methyl-2-phenylpiperazin-1-yl)pyridine-3-methanol was produced.

### Comparative example 1

One gram (1 g) of 2-(4-methyl-2-phenylpiperazin-1-yl)-3-cyanopyridine, 10 ml of an aqueous hydrochloric acid solution of 35% by weight, and 0.2 g of a 10% palladium-carbon catalyst were mixed and catalytically reduced in hydrogen at a hydrogen gage pressure of 3 kgf/cm² (294 kPa) and 70°C. After 5 hours, the reaction liquid was analyzed by a HPLC analysis, and it was confirmed that an objective product was hardly produced.
HPLC condition
Column: L-column ODS (5µm, 4.6 mmf×150 mm)
Mobile phase:
Liquid A: 1 mmol/L aqueous ammonium acetate solution (pH 5.75)
Liquid B: Acetonitrile
Concentration of Liquid B: 10% during 0 to 5 minutes in the retention time, 10% to 80% during 5 to 30 minutes, 80% during 30 to 40 minutes.
Flow rate: 1 ml/min
Column temperature: 40°C
Detected wavelength: UV220 nm

A production method of the present invention can efficiently produce 2-(4-methyl-2-phenylpiperazin-1-yl) pyridine-3-methanol useful as a production intermediate for mirtazapine without passing through a carboxylic acid derivative of the objective product, without requiring an anhydrous reaction system, and without using lithium aluminum hydride which is expensive.

## Claims

1. A method for producing 2-(4-methyl-2-phenylpiperazin-1-yl)pyridine-3-methanol which comprises catalytically reducing 2-(4-methyl-2-phenylpiperazin-1-yl)-3-cyanopyridine in the presence of a partially deactivated palladium catalyst in an aqueous acid solution.

2. The method according to claim 1, wherein the partially deactivated palladium catalyst is a palladium catalyst partially deactivated with a compound containing iron.

3. The method according to claim 2, wherein the compound containing iron is iron sulfate, iron chloride or iron acetate.

4. The method according to claim 1, wherein the acid aqueous solution is an aqueous sulfuric acid solution.

5. The method according to claim 4, wherein the partially deactivated palladium catalyst is prepared by adding a palladium catalyst of 1 to 10 parts by weight to an aqueous solution containing iron sulfate of 0. 05 to 0.1 part by weight, stirring the mixture at 0 to 40°C for 1 to 5 hours, filtrating and washing.

6. The method according to claim 1, wherein an amount of the partially deactivated palladium catalyst is 0.1 to 5 parts by weight as an amount of palladium metal based on 2-(4-methyl-2-phenylpiperazin-1-yl)-3-cyanopyridine of 100 parts by weight.
